# EUROPEAN PATENT SPECIFICATION

(11) **EP 1 561 468 B1**
(45) Date of publication and mention of the grant of the patent: **09.01.2008**
(21) Application number: 03730945.7
(22) Date of filing: 16.05.2003
(51) Int. Cl.: A61K 35/32, A61K 38/28, A61P 3/10

(54) **PHARMACEUTICAL ANTIDIABETIC COMPOSITION**
PHARMAZEUTISCHE ANTIDIABETES-ZUSAMMENSETZUNG
COMPOSITION PHARMACEUTIQUE ANTIDIABETIQUE

(30) Priority: 17.10.2002 RU 2002127819
(43) Date of publication of application: 10.08.2005
(73) Proprietor: Tsygankov, Vladimir Vladimirovich, Kaluzhskaya obl., 577298 (RU)
(72) Inventor: Tsygankov, Vladimir Vladimirovich, Kaluzhskaya obl., 577298 (RU)
(74) Representative: Tollett, Ian
(86) International application number: PCT/RU2003/000219
(87) International publication number: WO 2004/035069

(56) References cited:
- CN-A- 1 180 567
- CN-A- 1 284 364
- RU-C1- 2 054 292
- RU-C1- 2 059 411
- RU-C1- 2 077 887

## Description

### TECHNICAL FIELD

The present invention relates to pharmacology, medicine, particularly, to endocrinology, and provides a pharmaceutical antidiabetic composition.

### PRIOR ART

It is a common medical practice to use a large number of pharmaceutical preparations as antidiabetic medicaments. Such medicaments include insulin preparations extracted from bovine and porcine pancreas (insulin, suinsulin), as well as synthetic medicaments which have no hormonal action but are able to either stimulate secretion of insulin or inhibit destruction of the same (Mashkovsky M.D., Medicaments, in two volumes, 11th stereotype edition, Moscow, Meditsina Publishing House, 1988, p. 576).

The prior art preparations are characterized by high toxicity, multiple negative side effects and counter-indications.

Dietary supplements are known for administration directly with the meals to enrich the diet with biologically active substances or a combination thereof in case of various diseases. An example of such dietary supplement is the sunroot (Technical Conditions 9741-002-11866470-94) recommended in case of the diabetes mellitus, which comprises a concentrate of the natural biologically active substance prepared from the processed roots of a vegetable raw material, i.e. sunroot, the roots of which contain insulin, a vegetable substance having a hypoglycemic effect. However, the sunroot is effective as a hypoglycemic medicament in case of the II -type insulin-independent diabetes, while such dietary supplements are ineffective for treatment of the insulin dependent diabetes mellitus as in the latter case insulin has to be administered externally to the patients in the absence of their own insulin.

A powder-like biogenic preparation of hardened antlers is known from RU 2077887 A, which contains a complex of biologically active components of aminoacids, peptides, carbohydrate lipids, steroid hormones, fatty acids, phosphor-organic compounds, iodine, as well as a large number of micro- and macroelements (potassium, calcium, iron, magnesium, zinc, copper, manganese, nickel, tin, chromium, lithium, barium, and others). A variety of effects on the human organism is ensured by the original composition of the powder ingredients, such as enhancement of the adaptogenic resources, normalization of the metabolic effect, including correction of the lipid, protein and carbohydrate metabolism, regulation of the oxidation-reduction processes, hematosis, enhancement of the immunobiological and body defenses, stimulation of the growth and development of musculoskeletal tissues, elimination of heavy metals, toxins in humans, etc.

Said biogenic preparation with conserved natural activity of the original substance has a wide range of pharmacological effects not completely understood, so far. For this reason, it is not used as an antidiabetic medicament.

### SUMMARY OF THE INVENTION

It is the object of the present invention to use the prior art preparation of hardened antlers for a novel purpose as a medicament for treatment and prophylaxis of diabetes by means of revelation of its hypoglycemic effect.

To achieve said object a pharmaceutical antidiabetic composition is provided, characterized in that it comprises a powder extracted from hardened antlers, which contains 0.5 ng of insulin and 18 ng of insulin-like growth factor-I per 1 g of the composition.

Thereby, the use of said pharmaceutical composition as an antidiabetic medicament is made possible.

### DETAILED DESCRIPTION OF THE PREFERRED EMBODIMENT

The content of insulin and insulin-like growth factor-1 (IGF-I) has been determined by the ion-exchange chromatography method with a sufficiently high degree of purification.

180 g of the preparation in the form of a powder prepared from hardened antlers was extracted for 4 hours with simultaneous agitation in 20 volumes of the chloride ethanol solution. The resulting suspension was centrifuged and a supernatant was prepared, the pH of which was adjusted to 2.3 and which was kept at +4°C.

The resulting supernatant was applied on a column filled with the strong-acid cation-exchange resin in the H⁴" form. At the subsequent purification stages the protein-cationite complex was degreased with 70% ethanol, washed free from impurity proteins (with 0.5 M ammonium acetate solution), and insulin was eluted (with 0.2 N ammonium buffer solution). A spectrophotometer was used to control the composition of eluates at the wavelength of 280 nm. The protein peak detectable under the same conditions as that of the standard insulin was collected, and after adjusting its pH to 2.3 was subject to the first re-chromatography, and following eluate lyophilization, to the second re-chromatography. Following the three successive ion-exchange chromatography processes an eluate solution (measured at 280 nm) was obtained with the maximum protein concentration of 0.66 mg/ml.

After completion of the second and the third chromatography processes, isoelectric settling was conducted in the eluates. The impurity proteins associated with insulin were settled by adjusting the solution pH to 2.0 in a stepwise manner and after adding acetone (20 volume % of solution) - to 4.0. The mixture was kept for 1 hour at room temperature, and was left to stand overnight at +4°C. Then, the solution was centrifuged and the residual was removed.

In the process of settling zinc-IPP complexes, a 10%-solution of zinc acetate (2 volume %) was added to the supernatant, and its pH was adjusted to 5.95. The solution was kept at room temperature for 1 hour and subsequently placed in a fringe for 72 hours. The residual was separated by centrifugation.
Two specific radioreceptor systems were used to perform a radioligand assay of the extracted peptide material as to its capacity to bind with the insulin receptor and IGF-I receptor. Each system comprised a labeled hormone, tissue plasma membranes and standard non-labeled hormones. Crystal porcine insulin and human IGF-I were labeled by chloramine-¹²⁵Nal method. The measured specific activity of ¹²⁵I-insulin and ¹²⁵I-IGF-I constituted 160-180 millicurie/mg in both preparations.

Plasma membranes extracted from the liver of rats using Neville method (at 42.3% rate of rise of saccharose, with 15-20-time degree of membrane enrichment with receptors) were used in the insulin test system. Plasma membranes were used in the IGF-I test system extracted from the cerebrum of rats with an elevated content of receptors of this hormone (20000 g fraction with less than 10% of ¹²⁵I-IGF-I specific binding).

Therefore, the inventive composition includes components, which form an insulin-competent system balanced in a certain manner. It is by this reason that the insulin activity of the composition is explained, so that it became possible to use the same for prophylaxis treatment of the insulin-dependent and insulin-independent diabetes mellitus.

Tolerance and therapeutic efficiency of the powder was studied in the patients suffering from the low and medium severity insulin-independent diabetes with the associated adiposity.

40 patients suffering from the low and medium severity insulin-independent diabetes were kept under observation. A control group consisted of 20 patients receiving basic therapy. The patients were from 16 to 65 years old, all of them suffering from the low and medium severity insulin-independent diabetes. The exception was for the patients suffering from acute chronic diseases or exacerbation of chronic diseases.

400 mg (one unit-dose package) of the inventive composition per day was administered to the patients suffering from the low severity insulin-independent diabetes, 800 mg (2 unit-dose packages) - in case of the medium severity diabetes. The powder was admixed to the food eaten for breakfast and supper. The clinical tests lasted for 21 days.

All patients received a traditional course of treatment which included a calorie-reduced diet (the degree of reduction varied from 1200 kcal to 1500 kcal per day depending on the severity of disease), exercise therapy, hydro- and physiotherapeutic procedures.

All patients have had a verified diagnosis of the low and medium severity insulin-independent diabetes for 1 to 8 years.

The following indices were assessed in the course of treatment: blood sugar, cholesterol, thyroglobulin, total protein, uric acid , bilirubin, AST, ALT, prothrombin, fibrinogen, fibrinolysis time, Na, K. Ca, Mg, Fe, Zn, Cu. As the diagnosis was verified, no exercise tolerance test with glucose was performed.

The clinical tests were conducted in the following groups of patients:
1. The experimental group consisting of patients suffering from the medium severity insulin-independent diabetes - 20 persons (8 men and 12 women) at the age of 36 to 65 years, Kegle index - from 28 to 42.2 kg/m²;
2. The experimental group consisting of patients suffering from the low severity insulin-independent diabetes - 20 persons (9 men and 1 women) at the age of 36 to 60 years, Kegle index - from 32 to 42 kg/m²;
3. The control group consisting of patients suffering from the low and medium severity insulin-independent diabetes, who received the basic therapy not including the inventive composition, was similar to the experimental group as to the age-sex composition and clinical semiotics - 20 persons (8 men and 14 women at the age of 32 to 63 years) Kegle index - from 32,1 to 43.7 kg/m².

Disorders of the carbohydrate metabolism aggravated by adiposity of stages I-III were observed in the patients from all groups. The following associated diseases were also detected: hypertension (70% in the experimental group and 65% in the control group), polyosteoarthrosis deformans and osteochondrosis vertebrarium (76% and 72%, respectively), chronic cholecystitis in a state of remission (30% and 35%), large intestine dyskinesia of hypokinetic type with the constipation syndromes (70% and 75%).

**Table 1**

| Changes in the clinical indices of the II -type insulin-independent diabetic patients receiving treatment including the preparation of hardened antlers | | | | | | |
|---|---|---|---|---|---|---|
| Symptoms | Experimental group | | | | Control group | |
| | Medium severity | | Low severity | | Medium severity | Low severity |
| | 1 | 2 | 1 | 2 | 1 | 2 |
| Asthenia | 17(85%) | 3(15%) | 11(55%) | 0 | 16(80%) | 4(20%) |
| Fatigability | 17(80%) | 3(15%) | 11(55%) | 0 | 15(75%) | 2(10%) |
| Xerostomia | 15(75%) | 2(10%) | 7(35%) | 0 | 15(75%) | 2(10%) |
| Skin itch | 12(60%) | 0 | 7(35%) | 0 | 12(60%) | 1(5%) |
| Polydipsia | 7(35%) | 0 | 3(6%) | 0 | 6(30%) | 1(5%) |
| Hydruria | 7(35%) | 0 | 0 | 0 | 5(25%) | 0 |

The disease pattern before the treatment was rather typical for the patients belonging to all compared groups. The diabetic symptoms intensity depended on the severity of disease (Table 1).

**Table 2**

| Changes in clinical and biochemical indices (1 - before treatment. 2 - after treatment, * at p <0.05) | | | | | | |
|---|---|---|---|---|---|---|
| Indices | Experimental group | | | | Control group | |
| | Medium severity | | Low severity | | | |
| | 1 | 2 | 1 | 2 | 1 | 2 |
| Cholesterol, mM/l | 6.6+0.3 | 5.4+0.2* | 6.5+0.6 | 5.7+0.2 | 6.8+0.2 | 5.8+0.7 |
| Triglycerides, mM/l | 1.9+0.1 | 1.4+0.2* | 1.6+0.3 | 1.6+0.2 | 1.9+0.1 | 1.5+0.1 |
| Glucose, mM/l | 9.4+0.3 | 7.7+0.4* | 7.0+0.8 | 6.1+0.5 | 7.9+0.6 | 7.5+0.4 |
| Total protein, g/l | 83.6+0.6 | 79.8+0.3 | 79.7+0.5 | 78.3+0.5 | 78.3+0.9 | 76.5+0.8 |
| Uric acid, mcM/l | 0.28+0.02 | 0.25+0.02 | 0.26+0.02 | 0.24+0.02 | 0.28+0.01 | 0.26+0.02 |
| Bilirubin, mcM/I | 19.4+0.3 | 17.2+0.5 | 17.8+0.6 | 16.3+0.6 | 22.6+0.9 | 21.3+0.8 |
| ALT, RVU/I | 38.8+1.7 | 32.7+1.6 | 39.9+1.3 | 36.3+1.1 | 28.3+2.0 | 23.5+1.6 |
| AST, RVU/I | 32.2+1.7 | 26.9+1.5 | 29.0+0.9 | 23.7+0.7 | 21.8+1.9 | 20.3+4.0 |
| Prothrombin, % | 91.8+0.6 | 87.5+0.5 | 88.1+0.8 | 82.7+0.9 | 86.4+0.9 | 83.7+0.8 |
| Fibrinogen, mg% | 383+2.7 | 284+2.9 | 279+2.2 | 259+1.9 | 317+3.8 | 265+2.9 |
| Fibrinolysis time, min | 215+1.9 | 178+1.9 | 176+1.6 | 161+1.5 | 227+2.0 | 189+2.2 |
| Na, mM/l | 147+22.2 | 149+13.0 | 156+16.5 | 160+9.5 | 139+15.1 | 142+12.5 |
| K, mM/l | 4.4+0.1 | 4.3+0.1 | 4.4+0.5 | 4.9+0.5 | 4.5+0.6 | 4.6+0.4 |
| Ca, mM/l | 2.1+0.02 | 2.3+0.04 | 2.1+0.06 | 2.3+0.1 | 2.2+0.1 | 2.2+0.1 |
| Mg, mM/l | 1.4+0.1 | 1.3+0.2 | 1.0+0.1 | 1.1+0.4 | 1.3-0.2 | 1.2+0.1 |
| Fe, mcM/l | 30.0+1.4 | 30.7+4.3 | 26.7+9.0 | 36.0+3.6 | 30.1+3.2 | 31.0+5.4 |
| Zn, mcM/l | 20.5+3.1 | 20.7+3.2 | 19.1+5.5 | 19.2+4.6 | 20.5+4.2 | 20.9+3.9 |
| Cu, mcM/l | 17.8+1.4 | 19.2-, 1.7 | 20.7+2.7 | 21.5+3.3 | 21.3+3.2 | 22.1+2.1 |

As shown in Table 2. a rather high level of glycemia was observed in the medium severity diabetic patients. The changes in the carbohydrate metabolism indices show that there is a considerable decay of glucose content in the patients from the experimental group (by 20% in the medium severity diabetic patients, and by 12% in case of the low severity diabetes), while this index decreased only by 6% in the control group. The cholesterol content in blood decreased by 19% and 13%, respectively, in the medium and low severity diabetic patients from the experimental group, and by 15% in the patients from the control group.

As the state of mineral metabolism of the patients from the experimental group treated with the inventive composition was examined, a 10% increase of Ca content in the blood serum was observed as compared to the basic level, which is due to its high content in the composition.

**Table 3**

| Efficiency of treatment using the inventive composition | | | | |
|---|---|---|---|---|
| Groups of patients | Number of patients | Efficiency of treatment (number and % of patients) | | |
| | | High | Satisfactory | Poor |
| Experimental | 40 | 26(65%) | 14(35%) | 0 |
| Control | 20 | 6(30%) | 14(70%) | 0 |

The therapeutic effect of the treatment conducted in all compared groups was assessed as high, if the level of glycemia went down to the standard values, satisfactory, if a decrease of the blood sugar was rather significant but the blood sugar was still slightly in excess of the standard values, and poor, if no changes in the index have been observed. As shown in Table 3, normalization of the carbohydrate metabolism indices was observed in 65% of the insulin-independent diabetic patients suffering from associated adiposity, treated with the composition prepared from hardened antlers, against 30% in the control group.

The patient were completely satisfied with the organoleptic properties of the preparation, and noted its neutral taste, satisfactory appearance, thickness and color.

Not a single case of intolerance to the preparation, allergenic reactions or other side effects was observed over the period of conducting the clinical tests.

An effective therapeutic dose of the inventive pharmaceutical composition constitutes 400 mg per day (1 one unit-dose package) in case of the low seventy insulin-independent diabetes, and 800 mg (two unit-dose packages) in case of the medium severity insulin-independent diabetes.

Moreover, the preparation was tested for efficiency against clinical manifestations of the destructive tuberculosis in the patients also suffering from the low and medium severity insulin-independent diabetes, and positive results were obtained.

The level of glycemia was brought down to the standard value in six of 10 patients from group 1 suffering from the destructive tuberculosis in combination with the insulin-independent diabetes, who were treated with said preparation beside the combined chemotherapy, and in three of 10 patients from group 2 receiving only chemotherapy.

Relief of the diabetic clinical manifestations is a prerequisite for treating the principal disease in case of patients suffering from the destructive tuberculosis in combination with the insulin-independent diabetes. Therefore, owing to the hypoglycemic effect of the inventive preparation on the patients of this group, efficiency of chemotherapy of such patients may be increased.

It was proved that 400 to 800 mg of the inventive preparation administered per day produce a hypoglycemic effect on the insulin-independent diabetic patients so that they are able to reduce the dose of administered insulin.

The efficiency of the inventive composition is also proved with the below examples.
1. Patient P, 68. Suffers from the medium severity insulin-independent diabetes. Fasting blood sugar - 16.2 mmole/l.A one-month course of treatment was conducted, 0.8 g (two 0.4 g capsules) once a day. Blood sugar after administration - 6.48 mmole/l Simultaneous relief of the associated diabetic symptoms was observed: absence of thirst, normalization of sleep, skin itch remission, dysorexia.
2. Patient K. Has been suffering from the insulin-independent diabetes for eight years. After administration of two capsules of the preparation for two months, the blood sugar went down from 16 mmole/l to 7 mmole/l.
3. Patien 1, 41. Suffers from the medium severity insulin-independent diabetes. Fasting blood sugar before the administration of the preparation - 20 mmole/l, blood pressure - 170/110. After administration of two capsule of the preparation per day for two weeks, the blood sugar decreased by 9 units, the blood pressure became normal - 130/90.

Besides, clinical tests were conducted to determine the influence of the preparation on the plasma glucose in healthy humans. It was found that the preparation has no hypoglycemic effect when administered to healthy humans.

Therefore, as proved by the biochemical and clinical studies, the pharmaceutical composition prepared from hardened antlers has a pronounced insulin activity, and thereby produces a prophylactic and therapeutic effect when used for treating the diabetes mellitus.

### INDUSTRIAL APPLICABILITY

The present invention is suitable for prophylaxis and treatment of the insulin-dependent and insulin-independent diabetes mellitus. Besides, the preparation according to the invention is suitable for treatment and prophylaxis of the possible complications of the principal disease (diabetes mellitus): angiopathies including various trophic disturbances, retinopathies, various neuromuscular pathologies. It is a wide variety of elements the preparation comprises, which enables to normalize the lipid, protein and carbohydrate metabolism, improve the hemorheology, influence positively the state of vascular bed, improve the quality of neuromuscular transmission, make up for a deficiency in minerals.

## Claims

1. A pharmaceutical antidiabetic composition **characterised in that** it comprises a powder extracted from hardened antlers, which contains 0.5 ng of insulin and 18 ng of insulin-like growth factor-I per 1 g of the composition.

2. A dosage form comprising from 400 mag to 800 mg of the composition as claimed in claim 1 for decreasing blood sugar.

## Patentansprüche

1. Pharmazeutische Komposition der Antidiabeteswirkung, **dadurch gekennzeichnet, dass** sie ein aus verknöcherten Hirschhörnern abgesondertes Pulver darstellt, welches Insulin in einer Menge von 0,5 ng und insulinähnlichen Wachstumsfaktor-I in einer Menge von 18 ng pro 1 g Komposition enthält.

2. Arzneimittelform, enthaltend von 400 mg bis 800 mg Komposition gemäss Anspruch 1 zur Blutzuckersenkung.

## Revendications

1. Composition pharmaceutique à action antidiabétigue, **caractérisée en ce qu'**elle est réalisée en forme d'une poudre qui a été exatraite des cornes ossifiées des cerfs et qui contient l'insuline en quatité de 0,5 ng et un facteur de croissance similaire à l'insuline -1 v en quantité de 18 ng par 1 g de composition.

2. Preparation médicinale contenant de 400 mg à 800 mg de composition, réalisée selon la revendication 1 destinée à réduire, dans le sang, la teneur en sucre.
